# EUROPEAN PATENT APPLICATION

(11) **EP 3 991 740 A1**
(43) Date of publication of application: **04.05.2022**
(21) Application number: 20204054.9
(22) Date of filing: 27.10.2020
(51) Int. Cl.: A61K 35/747, A61K 35/20, A61K 36/53, A61K 36/534, A61K 36/73, A61P 1/00, A61P 37/04

(54) **MULTIFUNCTIONAL NUTRACEUTIC COMPOSITION MODELING THE GASTROINTESTINAL MICROBIOTA AND THE IMMUNE SYSTEM RESPONSE**

(71) Applicant: Lietuvos Sveikatos Mokslu Universitetas, 44307 Kaunas (LT)
(72) Inventor: Bartkien , Elena, 44307 Kaunas (LT); Bernatonien , Jurga, 44307 Kaunas (LT); Grigas, Juozas, 44307 Kaunas (LT); Ivanauskas, Liudas, 44307 Kaunas (LT); Jak tas, Valdas, Kaunas (LT); L l , Vita, 44307 Kaunas (LT); Pautienius, Arnoldas, 44307 Kaunas (LT); Pud iuvelyt , Lauryna, 44307 Kaunas (LT); Ru auskas, Modestas, Kaunas (LT); Savickas, Ar nas, 44307 Kaunas (LT); Stankevi ius, Ar nas, 44307 Kaunas (LT); akien , Vytaut, 44307 Kaunas (LT); Zavistanavi i t , Paulina, 44307 Kaunas (LT)
(74) Representative: Zaboliene, Reda

(57) **Abstract**

The invention relates to a nutraceutical composition that models a gastrointestinal microbiota by reducing the populations of opportunistic and pathogenic microorganisms in the gastrointestinal tract and stimulating the immune response by significantly increasing the proportions of B lymphocytes and macrophages/monocytes and IgG concentration, as well as significantly reducing the levels of T cytotoxic lymphocytes and T helpers (CD3⁺) in the body. The composition includes dehydrated lactic acid bacteria, fermented and lyophilized bovine colostrum, dehydrated apple pomace, oregano extract, mint essential oil, and thyme essential oil.

## Description

### The field of the invention

The essence of the present invention is providing of a nutraceutical composition that models a gastrointestinal microbiota by reducing the populations of opportunistic and pathogenic microorganisms in the gastrointestinal tract as well as activates the response of the humoral immune system.

### Background of the invention'

Humans and animals , are constantly at risk of getting infected with microorganisms including bacteria, viruses, protozoa, and helminths. Many innate and adaptive immune responses exist, and the stimulation and enhancement of these responses can reduce the number of diseases and infections. Such stimulation can be achieved by using nutraceuticals containing components that enhance the natural defense response.

The present invention provides consumers with a possibility to choose a safe antimicrobial composition that simulates an immune response, as there is currently no equivalent in the market to this invention. By creating an unfavourable environment for the multiplication of pathogenic and opportunistic microorganisms in the intestine and modelling the composition of the microbiota, the nutraceutical composition increases the number of probiotic and decreases the number of pathogenic and opportunistic bacteria in the intestine. This is important for the prevention of infectious diseases and the restoration of the natural intestinal microbiota after the use of antibiotics, antimicrobial agents or other oral substances and products that adversely affect the intestinal microbiota.

Various nutraceuticals have been found to have a significant effect on the intestinal (2) and respiratory (3) microbiota of the studied animals.

US 7553501 describes a composition for the treatment of immune diseases. The composition comprises specific formulations of plant extracts that have a synergistic effect and minimal side effects. The plant extracts mentioned are categorized into three groups important for human health depending on their effects: energy-giving, biologically advanced and improving well-being.

US 201662354287 describes a composition and methods for treatment or prevention of diseases and methods for preparing the composition. The composition consists of a protein component, a prebiotic component, and a probiotic component. The composition may be administered as a dietary supplement. Protein antibodies, immunoglobulins, glycoproteins are included as protein components into the composition. As a prebiotic, a yeast component is added to the composition; bacteria such as *Lactobacillus acidophilus, Lactobacillus brevis, Lactobacillus bulgaricus, Lactobacillus plantarum, Lactobacillus rhamnosus, Lactobacillus fermentum, Lactobacillus caucasicua, Lactobacillus helveticus, Lactobacillus lactis, Lactobacillus reuteri, Lactobacillus casei, Lactobacillus paracasei paracasei, Bifidobacterium bifldum, Bifidobacterium infantis, Bifidobacterium longum, Streptococcus thermophiles,* and *Streptococcus cremoris* are used as probiotics.

All published patent documents do not disclose a multifunctional nutraceutical composition formula that stimulates the gastrointestinal microbiota and immune response; the previous inventions have limited effects on the gastrointestinal microbiota and immune system.

The use of the invention would help to reduce the consumption of antibiotics, the development of antibiotic resistance side effects associated with the misuse of antibiotics, and the release of antibiotic residues into the environment. The unique, multifunctional formulation of six synergistically active components with a wide range of action in inhibiting pathogenic and opportunistic microorganisms has been developed.

Also, the present invention differs from the present ones in that the fruit/berry components of a multifunctional nutraceutical composition are derived from food industry by-products. Therefore, the process of production is more sustainable and matches with the principles of the circular economy.

Also, the present invention differs from the present ones in that all components of multifunctional nutraceutical composition are technologically functionalized to provide economic added value, to ensure biosafety and sustainability.

### Summary of the invention

The essence of the present invention is to provide a nutraceutical composition that models a gastrointestinal microbiota by reducing the populations of pathogenic microorganisms in the gastrointestinal tract and activates the response of the humoral immune system.

The composition consisting of ingredients with different mechanisms of action ensures the synergistic effect of an unfavourable environment for pathogenic and opportunistic microorganisms by inhibiting their multiplication in the intestine. The effect of the ingredients used alone is weaker compared to the composition.

The present invention provides a nutraceutical composition comprising at least the ingredients: whey-grown, lyophilized, and encapsulated lactic acid bacteria, fermented bovine colostrum in the presence *Lactobacillus plantarum* LUHS135, dehydrated apple pomace and essential oil.

*Lactobacillus* sp. strains of lactic acid bacteria (LAB), in particular strains of *Lactobacillus uvarum* and *Lactobacillus casei,* including but not limited to *Lactobacillus uvarum* LUHS245 and *Lactobacillus casei* LUHS210 (*Lactobacillus uvarum* strain 8 and *Lactobacillus casei* ATCC 334 strain) were used. The bacterial component grown in whey, lyophilized and encapsulated was prepared in a ratio (w/w) of 0.9 : 1 to 1 : 0.90, preferably 1 : 1. The concentration of viable LABs ranges from 6.0 to 9.0 logio CFU/g. The amount of the component is 1-10 g per day in the recommended dose.

Fermented in the presence of *Lactobacillus plantarum* LUHS135 (including but not limited to *Lactobacillus plantarum* strain JCM1149) and lyophilized bovine colostrum in an amount of 1 to 50 g per day at the recommended dose was used for the implementation of the invention.

Dehydrated apple pomace, in this case, of *Malus domestica* Borkh, in an amount of 1 to 50 g per day at the recommended dose was used.

The nutraceutical composition comprises a mixture of oregano herb extract, mint essential oil, and thyme essential oil. 100 g of the prepared mixture contains 98.950 g (not less than 94.750 g and not more than 99.000 g) of oregano herb liquid extract or preparation of the appropriate phytochemical composition, 0.312 g (not less than 0.296 g and not more than 1.560 g) of mint essential oil or preparation of the appropriate phytochemical composition and 0.738 g (not less than 0.701 g and not more than 3.690 g) of thyme essential oil or preparation of the appropriate phytochemical composition.

Menthol-type mint essential oil comprises essential oil obtained by various methods from above-ground parts of plants of the genus *Mentha* L. containing 0.8 mg/ml menthol or more.

Thymol-type thyme essential oil comprises essential oil obtained by various methods from flowering fresh above-ground parts of plants of the genus *Thymus* L. (including but not limited to *Thymus vulgaris* L., *T. zygis* L. or mixtures of parts of these species) containing 3 mg/mL thymol or more and 0.07 mg/ml carvacrol or more.

Oregano liquid extract comprises liquid extracts from flowering fresh above-ground parts of *Origanum* L. (including but not limited to *Origanum onites* L. or *Origanum vulgare* L. or mixtures of both) prepared by various methods using extraction solvents consisting of ethanol-water or non-polar solvents (including but not limited to glycerol, propylene glycol). Oregano herb extract containing 10 mg/ml carvacrol or more is the most suitable for the use. The best ratio of oregano extract, mint essential oil and thyme essential oil in the composition is 98.95 : 0.312 : 0.738. The amount of the component consisting of oregano herb extract, mint essential oil and thyme essential oil is 1-10 g per day in the recommended dose.

Another advantage of this composition is the inclusion of a component without any contraindication, i.e. fermented and dehydrated bovine colostrum, into the multifunctional formula. The latter component not only models the gastrointestinal microbiota but also has immune response modelling characteristics and is suitable for use in different age and high-risk consumer groups.

Also, the present invention differs from the present ones in manufacturing technology. The technology used for encapsulation of microorganisms with antimicrobial properties in nutraceutical composition was multiplication/encapsulation into by-products of the dairy industry. In this case, the production process is more sustainable and complies with the principles of the circular economy. The viability of microorganisms in the manufactured microcapsules is ensured during further production processes and storage.

### Brief description of the figures

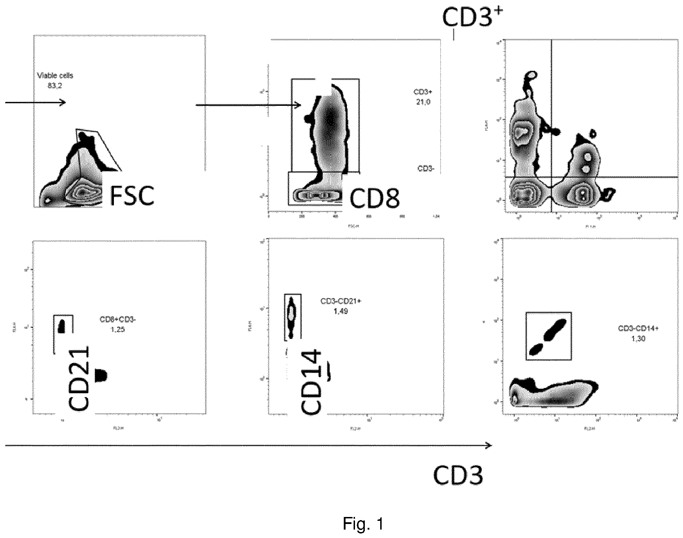

Fig. 1 Cytometric analysis data. (A) Viable cells were selected and sorted on FSC (linear light scattering) and SSC (lateral light scattering) to generate CD3⁺ and CD3⁻ cell regions. Other CD4-tagged and CD8-tagged regions were generated in the CD3⁺ region. (B) Regions based on CD3 and CD8, CD21, CD14 markers have been generated in the region of viable cells.

### A detailed description of the invention

A composition was developed wherein microbiota and immune response modelling properties were evaluated by methods applying *in vitro* and *in vivo* models.

Nutraceuticals are used for disease prevention, treatment, and immunomodulatory properties. Nutritional components such as lactic acid bacteria (LAB), bovine colostrum, apple by-products, and essential oils may positively alter the host's immune system and intestinal microbiota. However, their cumulative effect as a multicomponent nutraceutical has not been studied. Therefore, this study is the first wherein combination of LAB, bovine colostrum, dehydrated apple pomace, and essential oils was evaluated for their immunomodulatory, antimicrobial, and prebiotic properties in a pig model.

The composition consisting of ingredients with different mechanisms of action ensures the synergistic formation of an environment unfavourable for the multiplication of pathogenic and opportunistic microorganisms by inhibiting their activity in the intestine. The effect of single-use of the ingredients is weaker compared to the composition.

The composition comprises encapsulated dehydrated lactic acid bacteria (LAB) *Lactobacillus uvarum* LUHS245 + *Lactobacillus casei* LUHS210, *Lactobacillus plantarum* LUHS135, fermented and lyophilized bovine colostrum, dehydrated apple pomace, oregano herb extract, mint essential oil and thyme essential oil.

### Lactic acid bacteria

Probiotics are defined as living microorganisms that, when administered, have a positive effect on the health of the host. Despite the diversity of mechanisms of action, some probiotic strains have been shown to favour intestinal microbiota by relieving the symptoms of infectious diarrhoea, antibiotic-associated diarrhoea, irritable bowel syndrome (IBS), and other diseases of the gastrointestinal tract. Also, the immunomodulatory effects of probiotics have been demonstrated, making a positive impact on the conditions, such as atopic dermatitis, inflammatory bowel disease, rheumatoid arthritis, and autoimmune encephalomyelitis. In particular, the immunomodulatory properties of probiotic strains of the genus *Lactobacillus,* including inhibition of T helper 2 (Th2) cell-produced cytokines (IL-4 and IL-5), increase in CD4⁺ and CD8⁺ cell numbers, increase in IL-12 and INF -γ, and improvement in phagocytic activity. *L. casei* has been shown to increase the number of CD4, SWC3, CD11R1, and MHCII expressing mononuclear and dendritic cells in both porcine intestinal tissue and blood. To date, the effect of various *Lactobacillus* strains on the stability of faecal microbiota has been investigated, demonstrating a positive effect of probiotic strains on the smoothness index. In particular, *L. casei* subsp. *rhamnosus,* when used as a probiotic, has been associated with an increased abundance of Lactobacillaceae and Bifidobacteriaceae species, which is known to be useful in the treatment of gastrointestinal atopy, IBS, and other gastrointestinal disorders.

Encapsulated and dehydrated lactic acid bacteria (LAB) *Lactobacillus uvarum* and *Lactobacillus casei* strains grown in whey, lyophilized and encapsulated were used in the embodiment of the invention. *Lactobacillus uvarum* and *Lactobacillus casei* strains include, but are not limited to, *Lactobacillus uvarum* LUHS245 and *Lactobacillus casei* LUHS210 (*Lactobacillus uvarum* strain 8 and *Lactobacillus casei* strain ATCC 334).

The component consists of preform preparation containing strains of *Lactobacillus uvarum* and *Lactobacillus casei* in a ratio (w/w) of 1 : 1 (0.9 : 1 to 1 : 0.9). The concentration range of viable lactic acid bacteria in the component preform: of 6.0 to 9.0 logio CFU/g. The amount of component (g) per day in the recommended portion is 1-10 g. The present invention uses *Lactobacillus uvarum* LUHS245, deposited under accession No. 115308.1 and *Lactobacillus casei* LUHS210, deposited under accession No. 075032.1.

### Preparation of dehydrated lactic acid bacteria

Encapsulated and dehydrated lactic acid bacteria (LAB) *Lactobacillus uvarum* LUHS245 + *Lactobacillus casei* LUHS210 are prepared by propagating them in acid whey with 4.0% lactose, 0.8% protein, 0.5% lactic acid, 0.6% mineral substances, total dry matter 6.5%, and processing in a spray dryer. Before enrichment, acid whey was sterilized, cooled to 30 °C, and enriched with 2.5% glucose, 2.0% yeast extract, and 0.5% sucrose. Pure LAB was added in an amount of 3% (v/v) to the whey substrate and incubated under anaerobic conditions in a thermostat at 30 °C for 36 hours. Encapsulation was performed in a spray drying system (SD-06, Keison, GB). Propagated LAB have been injected peristaltically into the SD-06 system (inlet temperature 60 °C, inlet air temperature 150 °C, spray air temperature 100 °C, airflow 200 m³/h). The size of LAB capsules estimated by electron microscopy (Quanta SEM 200 FEG) was ≤10 µm. The viability of LAB in capsule powder was determined by the method of inoculation into Petri dishes of 8.9 CFU/g.

### Bovine colostrum

As a nutraceutical, bovine colostrum has adipogenic, antioxidant and anti-inflammatory properties associated with plenty of its biologically active molecules. Immunoglobulins, lactoferrin, lysozyme, antioxidants, oligopolysaccharides, proline-rich peptides, and cytokines are associated with antimicrobial and immunomodulatory effects. Bovine colostrum is rich in immunoglobulins, especially immunoglobulin G (IgG), which acts as a protective agent for the newborn calf. Recently, by stimulating a colostrum-producing cow with the desired antigen, hyperimmune bovine colostrum was obtained to produce high levels of colostrum immunoglobulins against the target antigen that was used as a preventive agent against pathogenic strains of *Clostridium, E. coli,* and *Shigella.* Lactoferrin is another biologically active component of colostrum that is responsible for a variety of immunomodulatory properties, including inhibition of anti-inflammatory cytokines and types I interferons, stimulation of morphological changes in dendritic cells, inhibition of T cell-produced cytokine synthesis, and increased amount of B cell antigens. Also, bovine colostrum contains prebiotic compounds such as fructooligosaccharides, galactooligosaccharides, gangliosides, and nucleosides that promote the growth of Bifidobacteriacea and Lactobacillales bacteria in the gut.

Fermented and lyophilized *Lactobacillus plantarum* (including but not limited to strain *Lactobacillus plantarum* JCM 1149) was used in the embodiment of the invention. The amount of component (g) per day in the recommended portion is 1-50 g.

### Preparation of fermented lyophilized bovine colostrum

It was used bovine colostrum taken immediately after calving (not later than 2 hours) of Lithuanian black and white Holstein dairy cows. Bovine colostrum was collected simultaneously and stored frozen at -20 ° C before technological functionalization. *Lactobacillus plantarum* LUHS135 used for fermentation of bovine colostrum before the experiment was stored at -80 ° C in a Microbank deep-freeze system (Pro-Lab Diagnostics, UK); LAB was propagated in MRS broth (CM 0359, Oxoid, Hampshire, UK) at 30 °C 48 hours in thermostat before the fermentation. LAB propagated in MRS broth of 3% (v/v) was inoculated into bovine colostrum and the mixture was fermented in a CO₂ atmosphere using Memmert GmbH + Co. KG incubator (Schwabach, Germany) for 24 hours at 30 °C. Cattle colostrum was lyophilized for 72 h at -40 °C (Lyophilizer 3 × 4 × 5, ZIRBUS Technology, Germany) at a condenser temperature of -85 °C and a pressure of 2 × 10⁻⁶ mPa following 24 hours of fermentation. A bulk product is obtained in powder form.

### Dehydrated apple pomace

Various phytochemicals, especially phenols and carotenoids, containing in vegetables and fruits have a positive health effect. Apple procyanidins have protective properties against gastrointestinal disorders such as inflammatory bowel disease and sensitization due to food allergies. Consumption of apple procyanidins alleviates the symptoms of inflammatory bowel disease in different immunomodulatory ways: by inhibiting IL-8 produced by intestinal epithelial cells, in turn by inhibiting the uptake of neutrophils into intestinal tissue and by reducing the production of INF-γ of CD4⁺ and CD4⁺ cells. Other phenols found in apples, such as condensed tannins, are also associated with immunomodulatory properties such as inhibition of T helper 17 cells, which in turn delays the development of rheumatoid arthritis. The positive effects of phenols and other biologically active compounds in apples were also associated with changes in the subject's microbiota that have been demonstrated in both *in vitro* and *in vivo* studies.

Dehydrated apple pomace, in this case, *Malus domestica* Borkh, in quantities of 1 to 50 g per day at the recommended dose was used for the embodiment of the present invention.

### Preparation of dehydrated apple pomace

"Auksis" apple pomace (a by-product of apple juice production) was dehydrated in a vacuum dryer at 45 ± 2.0 °C and a pressure of 6 × 10⁻³ mPa. Equipment XF020 (France-Etuves, France) was used in the process.

### Essential oils

Essential oils are known to have beneficial biological properties, including antimicrobial, antiviral, anti-inflammatory, and antioxidant effects. Most essential oils contain chemical compounds that belong to terpenoids and phenylpropanoids.

Oregano liquid extract includes liquid extracts prepared in a variety of ways using extraction solvents consisting of ethanol-water or non-polar solvents (including but not limited to glycerol, propylene glycol) from flowering fresh above-ground parts of *Origanum* L. (including but not limited to *Origanum onites* L. or *Origanum vulgare* L. or mixtures of both). Oregano is a rich source of active compounds such as polyphenols (flavonoids and phenolic acids), essential oils (with carvacrol and/or thymol, linalool, and p-cymene), triterpenoids, and sterols. This herb has a variety of health benefits, ranging from anti-inflammatory, antioxidant, antispasmodic to anti-urolithic, analgesic and antimicrobial effects.

Mint essential oil contains large amounts of menthol, menthone, 1,8-cineole, carvone and other compounds. Mint essential oil has been shown to be effective in treating IBS in humans. This effect is thought to be related to the antioxidant and anti-inflammatory effects of mint essential oil. Menthol-type mint essential oil comprises essential oil preparation obtained in various ways from the aboveground parts of plants of the genus *Mentha* L. Mint essential oil containing menthol in an amount of 0.8 mg/ml or more was used.

Thymol-type thyme essential oil includes essential oil preparation obtained in various ways from flowering fresh above-ground parts of plants of the genus *Thymus* L. (including but not limited to *Thymus vulgaris* L., *T. zygis* L. or mixtures of parts of these species).

*Thymus vulgaris* herb is a rich source of essential oil. The main compounds of thyme essential oil are carvacrol, thymol, geraniol, linalool and gamma-terpineol. Thyme essential oil containing thymol in an amount of 3 mg/ml or more and carvacrol in an amount of 0.07 mg/ml or more was used. The main components of thyme essential oil are responsible for their antimicrobial, antiviral, antioxidant, and anti-inflammatory properties. Thyme essential oil has a positive effect on the gastrointestinal environment. Thyme essential oil, which is dominated by thymol and carvacrol, increases pancreatic trypsin, lipase and protease activity, shows antispasmodic effects, reduces intestinal infections of gram-positive and gram-negative bacteria, fungi and yeasts, roundworms, ascarids, reduces oedema, various types of inflammation. Thyme essential oil can be used to treat ulcerative colitis and Crohn's disease.

In a mixture of oregano extract, mint essential oil and thyme essential oil, the best ratio is 98.95 : 0.312 : 0.738. The amount of component (g) per day in the recommended portion is 1-10 g.

### Preparation of oregano herb extract

Extraction by reflux was used to produce oregano herb extract. Place 1.0 g of the plant material in a 100 ml round-bottomed flask and add 100 ml of 90% vol ethanol or 90% ethanol/non-polar solvent mixture (glycerol, propylene glycol); extraction is carried out in a glycerol bath under reflux for 6 hours at 95 °C. The extract is filtered using 0.22 µm membrane filter for chromatographic analysis.

Oregano herb extract contains 10 mg/ml of carvacrol or more.

### Formulation of the composition

A composition is prepared to comprise the ingredients: whey-grown, lyophilized, and encapsulated lactic acid bacteria, fermented *Lactobacillus plantarum* LUHS135 bovine colostrum, dehydrated apple pomace and essential oils. The recommended amounts of the constituents of the composition to be consumed per day are given in Table 1.

**Table 1**

| Component definition | Amount of component (g) per day in recommended portion |
|---|---|
| The component consists of preform preparation containing *Lactobacillus uvarum* LUHS245 and *Lactobacillus casei* LUHS210 (*Lactobacillus uvarum* strain 8 and *Lactobacillus casei* ATCC 334 strain), proportion (w/ w): 1 : 1 (of 0.9 : 1 to 1 : 0.9). | 1-10 |
| Bovine colostrum fermented in the presence of *Lactobacillus plantarum* (including but not limited to strain *Lactobacillus plantarum* JCM 1149) and lyophilized | 1-50 |
| *Malus domestica* Borkh. fruit pomace, dehydrated | 1-50 |
| A mixture of oregano extract, mint essential oil, and thyme essential oil | 1-10 |

### Methods of preparation of a nutraceutical composition

A composition comprising ingredients: encapsulated and dehydrated LAB *Lactobacillus uvarum* LUHS245 + *Lactobacillus casei* LUHS210, fermented *Lactobacillus plantarum* LUHS135 lyophilized bovine colostrum, dehydrated apple pomace, oregano extract, mint essential oil, and thyme essential oil may be presented in solid form (tablets, sweets, such as gums, dragee, etc.); in liquid form (emulsions, cocktails, soft drinks and juices enriching with the above components, etc.); in powder form (by mixing the components in the amounts of the invention).

Innovative liquid/solid technology has been applied for improving the bioavailability of sparingly water-soluble active compounds (thyme and mint essential oils) and for use in powder production. The liquid/solid phase system technology was performed: using a simple mixing method, where the solution of active ingredients and excipients were mixed until the formation of suitable powder properties. Magnesium aluminium metasilicate (Neusilin) was chosen as the carrier for the production of the powder in the liquid/solid phase. The carrier and ethanolic oregano extract and the mint and thyme essential oils preparation were mixed in a mortar, sieved through a 1 mm sieve, and homogenized in a blender for 10 minutes. The prepared mixture was placed in a dryer at 60 °C for 1 hour, the procedure was repeated until all quantity of the solution has been used. The prepared mixture was placed in a ventilated dryer at 40 °C for 24 hours. After drying, a free-flowing, yellowish powder of specific smell was obtained. Chromatographic analysis showed that the amounts of the main biologically active compounds, thymol, carvacrol and menthol, were not significantly reduced in the powder formulations. A powder obtained by the liquid/solid phase technology was further used in the tabletting process. The resulting free-flowing and high-quality powder were tableted into mini-tablets, which were coated with a bilayer coating for distinct release in the gastrointestinal tract.

The tablets were covered using the apparatus Erweka AR 403 with a coating pan D63150. Shellac solution of 10% containing 2.8 oleic acid and 10 ml of 10% ammonia solution in 43% ethanol was used for coating. The weight of the coating was 5% of the total weight of the tablet.

The tablets were manufactured using an Erweka RTP-D8 eccentric tablet press; wherein press speed was 4 pcs, compression force 5N, tablet weight 332 mg.

The composition may be prepared as powder formulation, as a liquid or solid product in any of the forms: tablets, capsules, microcapsules, powders, emulsions, oral solutions.

Applying an *in vivo* biological model, studies of the developed product demonstrated that a multifunctional nutraceutical had an effect on microbiota changes and humoral immunity in piglets. Piglets of the experimental group showed a statistically significant effect on populations of B lymphocyte, monocyte, T memory cell and Tc cell populations after 30 days of product administration. The changes of Th cell and NK cell populations were not significant compared to the control group. The studies showed that the nutraceutical also influenced the changes in the microbiota: a statistically significantly higher number of probiotic bacteria was found in the experimental group of animals compared to the control group, which did not receive the nutraceutical composition.

The problem of formulation of the multifunctional composition was solved by using six technologically functionalized antimicrobial components with different modes of action, whose synergistic effect (mechanism of action of the invention) (I) in inhibition of pathogenic and opportunistic microorganisms effectively increases compared to single components or compositions consisting of 2 or 3 components; (II) the microorganisms used in the composition provide a favourable medium for the proliferation of probiotic bacteria naturally present in the gut, such as Bifidobacteriacea and Lactobacillales, but at the same time, the microorganisms used in the composition do not remain there for a long time. Thus, no additional competitive microbiota is introduced into the gut, it only provides a favourable medium for the proliferation of natural probiotic bacteria and at the same time for the activation of humoral immunity. Besides, the mechanism of action (III) is explained by the inclusion of a non-contraindicated component in a formulation that not only models the gastrointestinal microbiota but also has immune response activation characteristics and is suitable for use by different age and high-risk groups.

### Mechanism of action on pathogenic bacteria

The mechanism of action against pathogenic bacteria manifests itself in two ways: (1) antimicrobial components, directly and indirectly, inhibit the growth of pathogenic bacteria, and (2) nutraceutical, as a complex composition, regulates populations of individual groups of bacteria. In the first case, lactic acid bacteria form an acidic environment (low ambient pH) that is unfavourable for pathogenic bacteria to multiply. Also, lactic acid bacteria directly inhibit the expansion of pathogenic bacteria by secreting bacteriocins.

This has been demonstrated in *in vitro* studies investigating the inhibitory properties of lactic acid bacteria against various pathogenic bacterial pathogens. In the second case, *in vivo* studies have shown that the composition increases the percentage of probiotic bacteria in the gut, thus reducing the number of other bacteria, including pathogenic ones. This was determined by metagenomic studies of the bacterial profile, quantifying the relative and exact amounts of all bacterial groups in the intestinal samples taken.

The effects on pathogenic bacteria were investigated by the following methods: *in vitro* in solid media, *in vitro* in liquid media and *in vivo,* i.e. metagenomic bacterial profile study and quantification of the ratio and exact amount of all bacterial groups in intestinal samples were assessed.

*In vitro* studies have been performed to analyze the inhibitory effect on a wide range of gram-positive and gram-negative bacteria.

*In vivo* studies provided data on the ratios of all bacterial groups and their exact amounts in intestinal samples taken.

*In vitro,* all tested pathogenic opportunistic bacteria were found to be inhibited by the compositions presented in the application (in a solid medium by assessing the diameter of the inhibition zones, in a liquid medium by assessing pathogen viability).

The application of the invention would help to reduce the consumption of antibiotics, the development of antibiotic resistance of pathogens, and at the same time the release of antibiotic residues into the environment. A unique multi-functional formula of six synergistically acting components with a broad spectrum of inhibiting pathogenic and opportunistic microorganisms has been developed.

The developed prototype preparation would be a suitable means for reducing the use of antibiotics in some agricultural sectors, such as pig farming, where the resistance of microorganisms to antibiotics and the amount of antimicrobial preparations used has recently tended to increase (not only in Lithuania but also in other countries). The present invention can also make a significant contribution to the global strategy to mitigate climate change by addressing environmental issues, as the multifunctional nutraceutical composition uses efficient valorisation technologies for food by-products.

Similar problems with the use of a six-component nutraceutical composition have not been solved in the past, but there have been attempts to use probiotic preparations consisting of microorganisms multiplying in the gut and thus changing the proportions of the microbiota with the emergence of new microorganisms. *In vivo* studies of the developed product have shown that the microorganisms in the multifunctional composition, among other ingredients of the composition, not only create favourable conditions for the proliferation of probiotic bacteria already present in the intestine but also an unfavourable environment for the multiplication of pathogenic and opportunistic microorganisms. However, they do not reside in the intestine for a long time and do not artificially change the species composition of the microbiota. *Lactobacillus* sp. strains isolated in Lithuania and not applied by other researchers were used to develop the preparation, wherein strains act synergistically with other nutraceutical components in the preparation and significantly activate changes in the body's microbiota and humoral immunity.

Synergistic action of nutraceutical components inhibits pathogenic and opportunistic microorganisms in the body of the potential user, stimulates the proliferation of probiotic bacteria naturally present in the gut, and activates the cells responsible for humoral immunity. Modelling of the microbiota and the immune response reduces the probability of developing infectious diseases and diseases, the development of which may be due to the inadequate intestinal balance of the microbiota. Besides, the developed product can protect individuals whose diet consists of low-fibre foods from gastrointestinal dysbacteriosis. In patients treated with antibiotics, the product can restore the balance of the microbiota more quickly and thereby minimize the undesirable effects of antibiotics.

In patients taking antibiotics, the product can restore the balance of the microbiota more quickly and thus reduce the adverse effects of antibiotics.

Also, the developed composition or parts thereof can be used in veterinary medicine, solving similar animal health problems, and strengthening immunity, and at the same time reducing the use of antibiotics in the animal husbandry sector.

### Examples

### In vivo experiment

Sixteen 6-week-old white piglets with an initial weight of 18.1 ± 1.6 kg were tested at the Animal Research Center of the Lithuanian University of Health Sciences. The animals were divided into 4 groups of 4 piglets and housed in separate enclosures. Control groups 1 and 2 consisted of females and males, respectively, and experimental groups 3 and 4 consisted of females and males, respectively. All animals were fed a standard concentrated diet. Groups 3 and 4 additionally received 30 g of nutraceutical preparation in the form of tablets mixed with the essential oil in daily routine. The experiment was run for 4 weeks.

### Sampling

Whole blood and faecal samples were taken before specialized feeding and on days 7, 14, and 28 after the start of the study. All collected blood samples were used for IgA, IgG, and IgM concentration measurements and flow cytometric analysis, and faecal samples were used for microbiome analysis. The cytometric analysis showed that the composition significantly increased the proportions of B lymphocytes and macrophages/monocytes and IgG concentration, as well as significantly reduced the amount of T cytotoxic lymphocytes and T helper (CD3⁺) in the body (Fig. 1).

This study showed that supplementation of pigs with a multi-component diet resulted in a statistically significant reduction in the proportions of T cytotoxic and doubly positive (CD4⁺ CD8⁺ decreased slightly) cells in the CD3⁺ cell population at 28 DPI compared to the start of the experiment (0DPI). In contrast, compared to the start of the experiments, a statistically significant increase in the proportions of B cells (along with an increase in IgG concentration) and macrophage/monocyte cells was observed in the viable cell population at 28 DPI. In addition, changes in the bacterial composition of the intestinal microbiota in pigs fed multicomponent diets at the end of the experiment changed significantly, with a 1.78-fold increase of prebiotic strains (*Bifidobacterium, Streptococcus, Faecalibacterium*) compared to control animals (Table 2).

Differences in the bacterial composition of the intestinal microbiota (% from the total microbiota)

**Table 2**

| Bacterial genera | Experimental group | Control group |
|---|---|---|
| *Bifidobacterium* | 5.81 | 3.6 |
| *Streptococcus* | 7.3 | 2.77 |
| *Lactobacillus* | 0.67 | 1.3 |
| *Enterococcus* | 0.03 | 0.02 |
| *Faecalibacterium* | 0.77 | 0.47 |
| *Bacillus* | 0.03 | 0.01 |
| *Escherichia* | 0.04 | 0.09 |

This study demonstrates the positive effect of the nutritional formula used in this study on changes in the intestinal microbiota, facilitating an increase in probiotic bacterial strains and potential anti-inflammatory properties. Therefore, the combination of components used in this study could be used as a feed to enhance the proportion of beneficial intestinal microorganisms without protection against inflammatory processes.

## Claims

1. A nutraceutical composition **characterized in that** it comprises at least:
(a) encapsulated and dehydrated *Lactobacillus* sp. strain of bacteria;
(b) bovine colostrum;
(c) dehydrated apple pomace;
(d) essential oil preparation;
for use in modelling of gastrointestinal microbiota for reducing populations of pathogenic microorganisms in the gastrointestinal tract, and in the activating the humoral immune system response.

2. The nutraceutical composition for use according to claim 1, **characterized in that** *Lactobacillus* sp. strains are *Lactobacillus uvarum* LUHS245 and *Lactobacillus casei* LUHS210.

3. The nutraceutical composition for use according to claim 1, **characterized in that** the bovine colostrum is fermented with *Lactobacillus plantarum* LUHS135 bacteria and lyophilized.

4. The nutraceutical composition for use according to claim 1, **characterized in that** the essential oil is mint essential oil and thyme essential oil.

5. The nutraceutical composition for use according to claim 1, **characterized in that** further comprising plant extracts.

6. The nutraceutical composition for use according to claim 5, **characterized in that** the plant extract is oregano herb extract.

7. The nutraceutical composition for use according to claim 1, **characterized in that** said composition comprises excipients.

8. The nutraceutical composition for use according to any one of claims 1 to 7, **characterized in that** said composition is prepared to inhibit the growth of pathogenic bacteria in the gastrointestinal tract of the host.

9. The nutraceutical composition for use according to any one of claims 1 to 7, **characterized in that** said composition is prepared to regulate populations of bacterial groups by reducing the incidence of pathogenic and increasing populations of probiotic bacteria such as Bifidobacteriaceae and Lactobacillales in the gut.

10. The nutraceutical composition for use according to any one of claims 1 to 7, **characterized in that** said composition is formulated to stimulate an immune response by significantly increasing the proportions of B lymphocytes and macrophages/monocytes and the concentration of IgG.
